Europäisches Patentamt

European Patent Office

Office européen des brevets

(11) Publication number: **0 183 457 B1**

# EUROPEAN PATENT SPECIFICATION

(45) Date of publication of patent specification: **15.01.92**   (51) Int. Cl.⁵: **A61K 31/35**, A61K 47/00

(21) Application number: **85308367.3**

(22) Date of filing: **18.11.85**

The file contains technical information submitted after the application was filed and not included in this specification

(54) **Aqueous composition containing sodium cromoglycate.**

(30) Priority: **23.11.84 US 674282**

(43) Date of publication of application:
**04.06.86 Bulletin 86/23**

(45) Publication of the grant of the patent:
**15.01.92 Bulletin 92/03**

(84) Designated Contracting States:
**AT DE NL SE**

(56) References cited:
**DE-A- 3 021 170**
**FR-A- 2 230 358**
**FR-A- 2 290 196**
**US-A- 4 053 628**

**REMINGTON's PHARMACEUTICAL SCI-ENCES, 17th ed., 1985; pp. 1556, 1561**

(73) Proprietor: **FISONS plc**
**Fison House Princes Street**
**Ipswich Suffolk IP1 1OH(GB)**

(72) Inventor: **Clemente, Emmett**
**23 Loading Place Road**
**Manchester, MA 01944(US)**
Inventor: **Pfeifer, Henry**
**371 Howard Street**
**Northborough, MA 01532(US)**
Inventor: **Hon-Kin Lee, Vincent, c/o School of Pharmacy**
**University of Wisconsin, 425 North Charter Street**
**Madison, WN 53706(US)**
Inventor: **Robinson, Joseph, c/o School of Pharmacy**
**University of Wisconsin, 425 North Charter Street**
**Madison, WN 53706(US)**

(74) Representative: **Craig, Christopher Bradberry et al**
**Fisons plc 12 Derby Road**
**Loughborough Leicestershire LE11 0BB(GB)**

## Description

This invention relates to a novel formulation and to a method for its preparation.

United States patent No 4 053 628 discloses a number of aqueous formulations of sodium cromoglycate. One such formulation of sodium cromoglycate (which is also known as cromolyn sodium) is a clear sterile aqueous solution which has been sold for a number of years and is used, inter alia, in the treatment of allergic conditions of the eye and nose. This solution suffers from the disadvantage that it has to be applied frequently, eg every four hours, for maximum effect.

Prima facie a prolongation of action of a drug for the treatment of the eye or nose can be achieved by forming the drug into an ointment or cream or by making the solution viscous.

European patent application No 0 100 592 discloses an eye ointment formulation of sodium cromoglycate which requires less frequent application than the aqueous solution. However this formulation suffers from the disadvantage that the ointment can obscure the vision and is not suited for day time use.

Further formulations of sodium cromoglycate, eg those containing polyvinylacetate and an oleaginous base, are disclosed in Int. J. Pharmaceutics, 16, 163-70 (1983). The polyvinylacetate formulation of sodium cromoglycate disclosed is rapidly cleared from eye. The oleaginous base suffers from the disadvantage that it forms crusts around the eye and obscures the patient's vision.

United States patent No 4 053 628 also discloses a viscous sodium cromoglycate formulation including sodium carboxymethyl cellulose. However, the patent does not state which grade of sodium carboxymethyl cellulose was used or what the final viscosity of the solution was.

Sodium cromoglycate is a highly polar molecule and is generally thought to act topically and not to penetrate tissue with ease. It is therefore to be expected that a satisfactory prolongation of the action of the drug necessitates the retention of the drug in contact with the eye for a prolonged period, eg by use of a highly viscous base with a viscosity of more than 5,000 mPa.s.

Surprisingly we have found that a relatively low viscosity solution can provide an appropriate prolongation of action.

According to the invention we provide a sterile aqueous ophthalmic solution of sodium cromoglycate which has a viscosity of from 150 to 300 mPa.s.

The formulation is preferably an isotonic solution.

The particular viscosity of the solution will depend to some extent on the nature of the condition to be treated and on the target tissue. Thus the formulation has a viscosity of from 150 to 300 mPa.s, more preferably from 175 to 275 mPa.s and especially from 180 to 250 mPa.s.

Viscosities of the solutions may be determined at room temperature with a suspended level viscometer, e.g. an Ubbelhode viscometer. However, viscosities are preferably determined using a rotational viscometer, preferably a Rheomat 30 (Rheomat is a trademark), at a temperature of from 20 to 25°C, eg 23°C, at a shear rate of from 40 to 65s$^{-1}$, eg 56.7s$^{-1}$.

A suitable viscosity may be produced by incorporating in the solution a viscosity enhancing agent, eg, a cellulose derivative and in particular hydroxypropyl methylcellulose (HPMC). The amount of the viscosity enhancing agent required to achieve the desired viscosity will depend on the particular agent used and also on its molecular weight. We prefer to use HPMC having from 20 to 30% methoxy groups, from 4 to 12% hydroxypropoxyl groups, and a gelation temperature in aqueous media of between 45 and 90°C, e.g. between 50 and 80°C and especially of about 56°C. We particularly prefer to use HPMC containing from 27 to 30% of methoxyl groups and 4 to 7.5% of hydroxypropoxyl groups. We also prefer the HPMC to have a number average molecular weight $M_n$ of from 80,000 to 90,000, e.g, of 86,000. We further prefer the HPMC to be such that a 2% aqueous solution has a viscosity of 3,500 to 4,500, e.g, about 4,000, mPa.s when measured at a temperature of 20°C. The HPMC is preferably low in metal ions, particularly Mg$^{++}$ and Ca$^{++}$. We particularly prefer that HPMC to be Hydroxypropyl Methylcellulose 2906 (USP), which is sold as Premium Methocel F4M or Shinetsu 65SH.

We prefer to use up to 1.5%, eg, 0.5% to 1.2% and especially about 1%, w/w of HPMC in the solution.

The solution may be made isotonic with sodium chloride.

The solution is preferably clear, and should be sterile.

The solution may be put up in unit dosage form, in which case preservatives may be incorporated, but are generally not necessary. Alternatively and preferably the solution may be put up in multi-dose form. In general it will be necessary to incorporate one or more preservatives into multi-dose formulations to ensure that the formulation remains sterile after initial use.

The solution may contain up to 10%, preferably 0.25% to 7.5%, eg, 1 to 5%, especially 2% or 4%, w/w of sodium cromoglycate.

Suitable preservatives include mercurial compounds, eg, sodium 2-(ethyl mercuriothio) benzoate (e.g, 0.001 to 0.05% w/w) or a quaternary ammonium compound such as benzalkonium chloride, eg, in a concentration of up to 0.015% w/w, and preferably from 0.005 to 0.0135% w/w, especially 0.01% w/w. Alternatively or additionally the composition may contain ethylenediamine tetraacetic acid or a salt thereof, eg, the disodium salt thereof, in, for example, a concentration of from 0.01% w/w to 0.5% w/w, especially 0.05% to 0.1% w/w.

When a preservative is used sterilisation of the solution by autoclaving is generally impracticable because the preservative reacts with the viscosity enhancing agent and is effectively deactivated. Furthermore heating of cold solutions of cellulosic viscosity enhancing agents, eg, HPMC, tends to cause the agent to precipitate out of solution (as the external parts of the solution heat up) and to form a heat insulating shield around the inside of the container. This shield makes the autoclaving process difficult and lengthy and may also cause some degradation of the product, e.g. as evinced by a change in the viscosity on cooling. The usual alternative to autoclaving, ie, sterile filtration of the finished product, is not reasonably possible as the viscosity is too high.

We have now found a method of producing sterile aqueous solutions containing sodium cromoglycate, a cellulose derivative viscosity enhancer, eg HPMC, and a preservative, eg, benzalkonium chloride, which comprises mixing under sterile conditions a sterilised solution of the cellulose derivative, optionally containing sodium chloride, with a sterile, eg, sterile filtered, solution containing preservative(s) and the sodium cromoglycate.

The solutions are preferably of equal weight and the concentration of the various components in each sufficient to give the concentrations desired in the final product.

The solution of the preservative(s) and the sodium cromoglycate may be made up simply by dissolving the ingredients in water which is low in metal ions.

The sterilised solution of the cellulose derivative may be made by dispersing the HPMC, eg, in powder form, in a hot aqueous solution of the sodium chloride followed by autoclaving, eg, at about 120°C for 15 minutes. The solutions of the invention may be put up in conventional unit dose or multi-dose containers.

The dosage to be administered will of course vary with the condition to be treated, with its severity and with its location. However, in general, a dosage of about 1 or 2 drops (eg, from 0.66 to 1.32mg of active ingredient) into the affected eye from 1 to 3 times a day is found to be satisfactory. More frequent dosage may, of course, be used if desired.

Conditions of the outer eye in which the compositions may be used include vernal catarrh (vernal kerato-conjunctivitis) and marginal corneal ulceration or infiltration. Other conditions which may be treated by the method of the invention include the ocular effects of hay fever, 'allergic eyes' where the allergen is known or unknown and spring/summer conjunctivitis. This latter term is used to mean allergic disorders of the eyes occurring in the spring and summer where an external allergen plays a part in the disorder. Further conditions of the eye which may be mentioned are 'irritable eye' or 'non-specific conjunctivitis', dry eye syndrome, Herpes Simplex Keratitis and Conjunctivitis, Herpes Zoster Keratitis and Conjunctivitis, adenovirus infections, phlyctenular conjunctivitis, corneal homograft rejection, Trachoma, anterior uveitis, anterior eye chamber disorders and drug sensitivity.

The formulations according to the present invention are advantageous in that they are longer acting, more acceptable to the patient, give rise to higher concentrations of sodium cromoglycate in target tissues, give rise to effective concentrations of sodium cromoglycate in target tissues for a longer time or are more stable than known similar formulations of sodium cromoglycate.

The invention is illustrated by the following examples.

## Example 1

### Ophthalmic Solution

| | |
|---|---|
| Sodium cromoglycate | 2% w/w |
| HPMC grade 2906 (USP) | 0.975% w/w |
| Benzalkonium chloride | 0.01% w/w |
| Ethylenediamine tetraacetic acid disodium salt (EDTA) | 0.1% w/w |
| Sodium hydroxide | q.s to pH 6.8 |
| Sodium chloride | 0.749% w/w |
| Purified water BP low metals to 100% | |

The solution was made by adding cold sterile sodium cromoglycate solution containing the benzalkonium chloride, the EDTA and the sodium hydroxide aseptically to an equal weight of hot sterile saline HPMC containing sodium chloride suspension with continuous mixing until the mixture was clear and homogeneous.

The solution was then filled aseptically in to a plastic dropper bottle of suitable capacity.

The solution was found to have a viscosity of 200 mPa.s at 23°C at a shear rate of $56.7s^{-1}$ using a Rheomat 30 * rotational viscometer.

## Example 2

### Ophthalmic solution

| | |
|---|---|
| Sodium cromoglycate | 2% w/w |
| HPMC grade 2906 | 0.95% w/w |
| Benzalkonium chloride | 0.01% w/w |
| Ethylenediamine tetraacetic acid disodium salt (EDTA) | 0.1% w/w |
| Sodium hydroxide | q.s. to pH 6.8 |
| Purified water BP low metals | to 100% |

The solution was made using the method of Example 1 and then filled aseptically into a plastic dropper bottle of suitable capacity.

The solution was found to have a viscosity of 200 mPa.s at 23°C at a shear rate of $56.7s^{-1}$ using a Rheomat 30 * rotational viscometer.

**Claims**

**Claims for the following Contracting States : DE, NL, SE**

*Rheomat 30 is a trademark

1. A sterile aqueous ophthalmic solution of sodium cromoglycate which has a viscosity of from 150 to 300 mPa.s.

2. A solution according to Claim 1, which has a viscosity of less than 275 mPa.s.

3. A solution according to any one of the preceding claims which has a viscosity of less than 250 mPa.s.

4. A solution according to any one of the preceding claims comprising hydroxypropyl methylcellulose.

5. A solution according to Claim 4 wherein the hydroxypropyl methylcellulose contains from 20 to 30% methoxy groups, from 4 to 12% hydroxypropoxyl groups and has a gelation temperature in aqueous media of between 45° and 90° C.

6. A solution according to Claim 4 or Claim 5, wherein the hydroxypropyl methylcellulose has a number average molecular weight of from 80,000 to 90,000.

7. A solution according to any one of Claims 4, 5 or 6 wherein the hydroxypropyl methylcellulose is such that a 2% aqueous solution has a viscosity of 3,500 to 4,500 mPa.s.

8. A solution according to Claim 4 comprising 0.5 to 1.2% w/w of hydroxypropyl methylcellulose.

9. A solution according to any one of the preceding claims comprising 0.25 to 7.5% w/w of sodium cromoglycate.

10. A solution according to any one of the preceding claims comprising up to 0.015% w/w of benzalkonium chloride.

11. A solution according to any one of the preceding claims comprising from 0.01 to 0.5% w/w of ethylenediamine tetraacetic acid or a salt thereof.

12. A solution according to any one of the preceding claims which is isotonic.

13. A method of making a sterile aqueous ophthalmic solution of viscosity/50 to 300 mPa.s containing sodium cromoglycate, a cellulose derivative viscosity enhancer and a preservative, which comprises mixing under sterile conditions a sterilised solution of the cellulose derivative with a sterile solution containing the preservative and the sodium cromoglycate.

**Claims for the following State : AT**

1. A method of making a sterile aqueous solution of sodium cromoglycate which has a viscosity of from 150 to 300 mPa.s, which comprises mixing an aqueous solution of sodium cromoglycate with a viscosity enhancer.

2. A method according to Claim 1, wherein the solution has a viscosity of less than 275 mPa.s.

3. A method according to any one of the preceding claims, wherein the solution has a viscosity of less than 250 mPa.s.

4. A method according to any one of the preceding claims, wherein the viscosity enhancer comprises hydroxypropyl methylcellulose.

5. A method according to Claim 4 wherein the hydroxypropyl methylcellulose contains from 20 to 30% methoxy groups, from 4 to 12% hydroxypropoxyl groups and has a gelation temperature in aqueous media of between 45° and 90° C.

6. A method according to Claim 4 or Claim 5, wherein the hydroxypropyl methylcellulose has a number average molecular weight of from 80,000 to 90,000.

**7.** A method according to any one of Claims 4, 5 or 6 wherein the hydroxypropyl methylcellulose is such that a 2% aqueous solution has a viscosity of 3,500 to 4,500 mPa.s.

**8.** A method according to Claim 4, wherein the solution comprises 0.5 to 1.2% w/w of hydroxypropyl methylcellulose.

**9.** A method according to any one of the preceding claims, wherein the solution comprises 0.25 to 7.5% w/w of sodium cromoglycate.

**10.** A method according to any one of the preceding claims, wherein the solution comprises up to 0.015% w/w of benzalkonium chloride.

**11.** A method according to any one of the preceding claims, wherein the solution comprises from 0.01 to 0.5% w/w of ethylenediamine tetraacetic acid or a salt thereof.

**12.** A method according to any one of the preceding claims, wherein the solution is isotonic.

**Revendications**
**Revendications pour les Etats contractants suivants : DE, NL, SE**

**1.** Solution ophtalmique aqueuse stérile de cromoglycate sodique qui a une viscosité de 150 à 300 mPa.s.

**2.** Solution suivant la revendication 1, qui a une viscosité de moins de 275 mPa.s.

**3.** Solution suivant l'une quelconque des revendications précédentes, qui a une viscosité de moins de 250 mPa.s.

**4.** Solution suivant l'une quelconque des revendications précédentes, comprenant de l'hydoxypropylmé-thylcellulose .

**5.** Solution suivant la revendication 4, dans laquelle l'hydroxypropylméthylcellulose contient 20 à 30% de radicaux méthoxy, 4 à 12% de radicaux hydoxypropoxy et a une température de gélification en milieu aqueux entre 45 et 90° C.

**6.** Solution suivant la revendication 4 ou 5, dans laquelle l'hydoxypropylméthylcellulose a un poids moléculaire moyen en nombre de 80 000 à 90 000.

**7.** Solution suivant la revendication 4, 5 ou 6, dans laquelle l'hydroxypropylméthylcellulose est telle que qu'une solution aqueuse à 2% ait une viscosité de 3500 à 4500 mPa.s.

**8.** Solution suivant la revendication 4, comprenant 0,5 à 1,2% p/p d'hydroxypropylméthylcellulose.

**9.** Solution suivant l'une quelconque des revendications précédentes, comprenant 0,25 à 7,5% de cromoglycate sodique.

**10.** Solution suivant l'une quelconque des revendications précédentes, comprenant jusqu'à 0,015% p/p de chlorure de benzalkonium.

**11.** Solution suivant l'une quelconque des revendications précédentes, comprenant 0,01 à 0,5% d'acide éthylènediaminetéraacétique ou d'un sel de celui-ci.

**12.** Solution suivant l'une quelconque des revendications précédentse, qui est isotonique.

**13.** Procédé de préparation d'une solution ophtalmique aqueuse stérile d'une viscosité de 150 à 300 mPa.s contenant du cromoglycate sodique, un dérivé cellulosique augmentant la viscosité et un conservateur, qui comprend l'action de mélanger, dans des conditions stériles, une solution stérilisée du dérivé cellulosique avec une solution stérile contenant le conservateur et le cromoglycate sodique.

**Revendications pour l'Etat contractant suivant : AT**

1. Procédé de préparation d'une solution ophtalmique aqueuse stérile de cromoglycate sodique qui a une viscosité de 150 à 300 mPa.s, qui comprend l'action de mélanger une solution aqueuse de cromoglycate sodique avec un agent augmentant la viscosité.

2. Procédé suivant la revendication 1, dans lequel la solution a une viscosité de moins de 275 mPa.s.

3. Procédé suivant l'une quelconque des revendications précédentes, dans lequel la solution a une viscosité de moins de 250 mPa.s.

4. Procédé suivant l'une quelconque des revendications précédentes, dans lequel l'agent augmentant la viscosité comprend de l'hydoxypropylméthylcellulose.

5. Procédé suivant la revendication 4, dans lequel l'hydroxypropylméthylcellulose contient 20 à 30% de radicaux méthoxy, 4 à 12% de radicaux hydoxypropoxy et a une température de gélification en milieu aqueux entre 45 et 90° C.

6. Procédé suivant la revendication 4 ou 5, dans lequel l'hydoxypropylméthylcellulose a un poids moléculaire moyen en nombre de 80 000 à 90 000.

7. Procédé suivant la revendication 4, 5 ou 6, dans lequel l'hydoxypropylméthylcellulose est telle que qu'une solution aqueuse à 2% ait une viscosité de 3500 à 4500 mPa.s.

8. Procédé suivant la revendication 4, dans lequel la solution comprend 0,5 à 1,2% p/p d'hydoxypropylméthylcellulose.

9. Procédé suivant l'une quelconque des revendications précédentes, dans lequel la solution comprend 0,25 à 7,5% de cromoglycate sodique.

10. Procédé suivant l'une quelconque des revendications précédentes, dans lequel la solution comprend jusqu'à 0,015% p/p de chlorure de benzalkonium.

11. Procédé suivant l'une quelconque des revendications précédentes, dans lequel la solution comprend 0,01 à 0,5% d'acide éthylènediaminetéraacétique ou d'un sel de celui-ci.

12. Procédé suivant l'une quelconque des revendications précédentse, dans lequel la solution est isotonique.

**Patentansprüche**
**Patentansprüche für folgende Vertragsstaaten : DE, NL, SE**

1. Sterile wässerige opthalmische Lösung von Natriumcromoglykat, die eine Viskosität von 150 bis 300 mPa.s aufweist.

2. Lösung nach Anspruch 1, die eine Viskosität von weniger als 275 mPa.s aufweist.

3. Lösung nach einem der vorhergehenden Ansprüche, die eine Viskosität von weniger als 250 mPa.s aufweist.

4. Lösung nach einem der vorhergehenden Ansprüche umfassend Hydroxypropylmethylzellulose.

5. Lösung nach Anspruch 4, worin die Hydroxypropylmethylzellulose von 20 bis 30 % Methoxygruppen, von 4 bis 12 % Hydroxypropoxylgruppen enthält und eine Geliertemperatur in wässerigen Medien von zwischen 45° und 90° C aufweist.

6. Lösung nach Anspruch 4 oder 5, worin die Hydroxypropylmethylzellulose ein Zahlenmittel des Molgewichts von 80 000 bis 90 000 aufweist.

**7.** Lösung nach einem der Ansprüche 4, 5 oder 6, worin die Hydroxypropylmethylzellulose derart ist, daß eine 2 %ige wässerige Lösung eine Viskosität von 3500 bis 4500 mPa.s aufweist.

**8.** Lösung nach Anspruch 4 umfassend 0,5 bis 1,2 % G/G Hydroxypropylmethylzellulose.

**9.** Lösung nach einem der vorhergehenden Ansprüche umfassend 0,25 bis 7,5 % G/G Natriumcromoglykat.

**10.** Lösung nach einem der vorhergehenden Ansprüche umfassend bis zu 0,015 % G/G Benzalkoniumchlorid.

**11.** Lösung nach einem der vorhergehenden Ansprüche umfassend von 0,01 bis 0,5 % G/G Ethylendiamintetraessigsäure oder ein Salz hievon.

**12.** Lösung nach einem der vorhergehenden Ansprüche, die isotonisch ist.

**13.** Verfahren zur Herstellung einer sterilen wässerigen opthalmischen Lösung mit einer Viskosität von 150 bis 300 mPa.s, enthaltend Natriumcromoglykat, einen Zellulosederivat-Viskositätsverstärker und ein Konservierungsmittel, welches Verfahren das Mischen unter sterilen Bedingungen einer sterilisierten Lösung des Zellulosederivats mit einer das Konservierungsmittel und das Natriumcromoglykat enthaltenden sterilen Lösung umfaßt.

**Patentansprüche für folgenden Vertragsstaat : AT**

**1.** Verfahren zur Herstellung einer sterilen wässerigen Lösung von Natriumcromoglykat, die eine Viskosität von 150 bis 300 mPa.s aufweist, welches Verfahren das Mischen einer wässerigen Lösung von Natriumcromoglykat mit einem Viskositätsverstärker umfaßt.

**2.** Verfahren nach Anspruch 1, wobei die Lösung eine Viskosität von weniger als 275 mPa.s aufweist.

**3.** Verfahren nach einem der vorhergehenden Ansprüche, wobei die Lösung eine Viskosität von weniger als 250 mPa.s aufweist.

**4.** Verfahren nach einem der vorhergehenden Ansprüche, wobei der Viskositätsverstärker Hydroxypropylmethylzellulose umfaßt.

**5.** Verfahren nach Anspruch 4, wobei die Hydroxypropylmethylzellulose von 20 bis 30 % Methoxygruppen, von 4 bis 12 % Hydroxypropoxylgruppen enthält und eine Geliertemperatur in wässerigen Medien von zwischen 45° und 90° C aufweist.

**6.** Verfahren nach Anspruch 4 oder 5, wobei die Hydroxypropylmethylzellulose ein Zahlenmittel des Molgewichts von 80 000 bis 90 000 aufweist.

**7.** Verfahren nach einem der Ansprüche 4, 5 oder 6, wobei die Hydroxypropylmethylzellulose derart ist, daß eine 2 %ige wässerige Lösung eine Viskosität von 3500 bis 4500 mPa.s aufweist.

**8.** Verfahren nach Anspruch 4, wobei die Lösung 0,5 bis 1,2 % G/G Hydroxypropylmethylzellulose umfaßt.

**9.** Verfahren nach einem der vorhergehenden Ansprüche, wobei die Lösung 0,25 bis 7,5 % G/G Natriumcromoglykat umfaßt.

**10.** Verfahren nach einem der vorhergehenden Ansprüche, wobei die Lösung bis zu 0,015 % G/G Benzalkoniumchlorid umfaßt.

**11.** Verfahren nach einem der vorhergehenden Ansprüche, wobei die Lösung von 0,01 bis 0,5 % G/G Ethylendiamintetraessigsäure oder ein Salz hievon umfaßt.

**12.** Verfahren nach einem der vorhergehenden Ansprüche, wobei die Lösung isotonisch ist.